# EUROPEAN PATENT APPLICATION

(11) **EP 3 861 996 A1**
(43) Date of publication of application: **11.08.2021**
(21) Application number: 20382072.5
(22) Date of filing: 04.02.2020
(51) Int. Cl.: A61K 31/352, A61K 9/00, A61K 9/06, A61K 47/36, A61P 29/00

(54) **CONTROLLED RELEASE SYSTEM OF PHYTOCANNABINOIDS FORMULATIONS SOLUBLE IN AQUEOUS MEDIA, METHODS AND USES THEREOF**

(71) Applicant: I+Med S. Coop., 01510 Vitoria- Gasteiz Alava (ES)
(72) Inventor: ALONSO CARNICERO, José, María, 01510 VITORIA- GASTEIZ (Álava) (ES); PÉREZ GONZÁLEZ, Raúl, 01510 VITORIA- GASTEIZ (Álava) (ES); SÁEZ MARTÍNEZ, Virginia, 01510 VITORIA- GASTEIZ (Álava) (ES); MUÑOZ MORENTIN, Manuel, 01510 VITORIA- GASTEIZ (Álava) (ES)
(74) Representative: Herrero & Asociados, S.L.

(57) **Abstract**

The present invention refers to a controlled release system of phytocannabinoids formulation soluble in aqueous media comprising phytocannabinoids, or derivatives thereof, a non-ionic surfactant and a cross-linked polymer, wherein the pH of the compositions ranges between 4 and 9, and wherein the weight ratio of cannabinoid/hyaluronic acid is 50:1 to 1:50, and the weight ratio cannabinoid/non-ionic surfactant is 1:30 to 1:1. The invention also refers to methods of producing the controlled release system of cannabinoids formulation soluble in aqueous medium and the pharmaceutical, cosmetic and nutraceutical applications thereof. The methods of the present invention allow the cross-linking of hyaluronic acid containing encapsulated or vehiculized phytocannabinoids at neutral pH, avoiding the degradation of the phytocannabinoids and preserving its functionality.

## Description

### Field of the invention

The present invention relates generally to formulations of phytocannabinoids. In particular, the invention relates to a controlled release system of phytocannabinoids formulation soluble in aqueous media, the method for the manufacture of this controlled release system and its uses in pharmaceutical, nutraceutical and cosmetic applications.

### Background of the invention

Phytocannabinoids have pharmacological activity as they interact with the central nervous system (psychotropic). However, only some of them are psychoactive and cause an alteration of perception, mood and can cause addiction.

Phytocannabinoid derivatives have a wide variety of therapeutic applications that can be divided according to different medical conditions:
- Neurological and psychiatric disorders: multiple sclerosis, spasticity, epilepsy, Parkinson's disease, Alzheimer's disease, anxiety, depression, stress, bipolar disorders,
- Digestive and eating disorders: anorexia, loss of appetite, nausea, diabetes, Crohn's disease,
- Pain and inflammation: headache, migraine, fibromyalgia, inflammation, arthritis, cramps, spinal cord injury, muscle spasms, sprains and muscle injuries. Treatment of pain of peripheral origin both chronic and acute (contusions, postoperative),
- Inflammatory skin conditions: acne, psoriasis, atopic dermatitis and treatment of conflicting wounds (bedsores, diabetic foot),
- Others: cancer, insomnia, lupus, hypertension, ischemia, muscular dystrophy, fatigue, glaucoma, asthma.

Phytocannabinoids dissolve easily in lipids, alcohols and other non-polar organic solvents, but display low water solubility. This fact limits its bioavailability and makes its encapsulation necessary in order to optimize its therapeutic activity and minimize its possible addictive properties.

Within the family of phytocannabinoids, the derivatives that have received the most attention are THC (tetrahydrocannabinol) that causes dependence, as well as CBD (cannabidiol) and CBG (cannabigerol) that are not addictive. In this sense, the US regulatory agency FDA (Food and Drug Administration) has approved several medications that contain THC and CBD derivatives in its composition: Marinol® (dronabinol) and Cesamet® (nabilone) that are synthetic analogs of tetrahydrocannabinol (THC) Epidiolex® which is a pure cannabidiol (CBD) extracted from the plant and Sativex® (nabiximols): cannabis extract with a 1: 1 ratio of THC and CBD. Of these medicines only Sativex has been approved by the EMA (European Medicines Agency) for use in the EU.

Cannabidiol (CBD) is the non-psychoactive analog of tetrahydrocannabinol (THC). From a pharmacological point of view cannabidiol has little binding affinity for either CB1 and CB2 receptor but is capable of antagonizing them in the presence of other phytocannabionoids such as THC. CBD also regulates the perception of pain by affecting the activity of a significant number of targets including non-phytocannabinoid G protein-coupled receptors, ion channels and peroxisome proliferator- activated receptor. CBD displays as well anti-inflammatory and anti-spasmodic benefits. Other phytocannabinoids that can contribute to the analgesic effects of CBD is for instance cannabigerol (CBG). Similarly to CBD, CBG does not display significant affinities for phytocannabinoid receptors but they have other modes of action.

Hyaluronic acid is a major component of the extracellular matrix (ECM) and thus is the major physiological constituent of the articular cartilage matrix and is particularly abundant in synovial fluid and in the skin.
The hyaluronic acid, in its acid or salt form, is a biomaterial broadly employed as an injectable material for applications in tissue engineering and especially for augmentation of skin tissue and of other soft tissues.

Hyaluronic acid is a linear non-sulfated glycosaminoglycan biopolymer composed of repeating units of D-glucuronic acid and N-acetyl-D-glucosamine (Tammi R., Agren U M., Tuhkanen A L., Tammi M. Hyaluronan metabolism in skin. Progress in Histochemistry & Cytochemistry 29 (2): 1.-81, 1994). At physiological pH (7.4) it is in the conjugate base hyaluronate form.

Hyaluronic acid is mostly synthesized in the skin by dermal fibroblasts and epidermal keratinocytes (*Tammi R., et al*, 1994) and acts as a water pump for maintaining the elasticity of the skin.

The ECM is composed of structural proteins such as collagen and elastin and of water, minerals and proteoglycans. This matrix is a dynamic structure with a structural role that provides to the skin with its mechanical properties of elasticity, firmness and tone.

Concerning the skin, it is noticed that, with age, the amount of hyaluronic acid and its degree of polymerization diminishes, causing a decrease in the amount of water retained in the connective tissue. In the meantime, ECM components are degraded, mainly by endopeptidase type enzymes.

Lastly, the decrease in cellular defenses increases damage and disorders induced by external stresses such oxidative stress. The skin is then subjected to an aging process leading to the appearance of defects and blemishes of keratinous substances, in particular of the skin.

Hydrogels of hyaluronic acid, and specifically based on crosslinked polymers, display numerous applications, especially as filling materials in traumatology, plastic and cosmetic surgery, ophthalmology and as products for preventing non-desired tissue adhesions. The applications indicated above for products of this type, without implying any limitation are familiar for those skilled in the art.

In the field of dermal fillers, gels, consisting mainly of hyaluronic acid, are injected intradermally to fill the skin wrinkles. Crosslinked hyaluronic acid allows a reduction of such wrinkles. However, it is known that the injection of such gels often produces a painful effect to the patient (US 2016/01 66554 A1).

Nowadays, in order to elude this technical problem, the main fillers based on hyaluronic acid are available with a local anesthetic agent to ensure greater patient comfort. This local anesthetic agent is only lidocaine, with a dosage of about 0.3%.

However, it is known that lidocaine may display the disadvantage, regarding its vasodilatory properties, to imply a too rapid absorption by the patient's body and sometimes an exacerbated occurrence of hematoma which has, for obvious aesthetic reasons, to be avoided as much as possible. On the other hand, phytocannabinoids, such as cannabidiol, are well known to exhibit pain relief, vasoconstriction and antiinflamatory effects. Therefore, they can be used to neutralize the side effects of the lidocaine or to diminish the inflammation provoke while injecting the soft tissue filler.

Joint diseases are injuries that affect human joints. Arthritis is the best known joint disease. Diseases of the joints may be variously short-lived or exceedingly chronic, agonizingly painful or merely nagging and uncomfortable; they may be confined to one joint or may affect many parts of the skeleton. Two principal categories are distinguished: inflammatory joint diseases in which inflammation is the principal set of signs or symptoms, and non-inflammatory joint diseases. Arthritis is a generic term for inflammatory joint disease. Regardless of the cause, inflammation of the joints may cause pain, stiffness, swelling, and some redness of the skin about the joint. Effusion of fluid into the joint cavity is common, and examination of this fluid is often a valuable procedure for determining the nature of the disease. The inflammation may be of such a nature and of such severity as to destroy the joint cartilage and underlying bone and cause irreparable deformities (WO2017203529A1).

Hyaluronic acid has been widely used for viscosupplementation of diseased or aged articular joints. However, recent investigations have revealed the active anti-inflammatory or chondroprotective effect of hyaluronic acid, suggesting its potential role in attenuation of joint damage (*Masuko, 2009* Masuko K, Murata M, Yudoh K, Kato T, Nakamura H, 2009, Anti-inflammatory effects of hyaluronan in arthritis therapy: Not just for viscosity. Int. J. General Medicine 2:77-81). Hyaluronan has been found to be effective in treatment of inflammatory processes in medical areas such as orthopedics, dermatology and ophthalmology, and it has been further found to be anti-inflammatory and antibacterial in gingivitis and periodontitis therapy.

Phytocannabinoids, and, in particular, cannabidiol (CBD), display anti-inflammatory properties. In this regard evidence exists that the effect of phytocannabinoids might be attenuation of the inflammatory component as occurs for example in rheumatoid arthritis (RA). Increasing evidence suggests that the endocannabinoid system, especially cannabinoid receptor 2 (CB2), has an important role in the pathophysiology of rheumatoid arthritis (RA). Many members of the endocannabinoid system are reported to inhibit synovial inflammation, hyperplasia, and cartilage destruction in RA. In particular, specific activation of CB2 may relieve RA by inhibiting not only the production of autoantibodies, proinflammatory cytokines, and matrix metalloproteinases (MMPs), but also bone erosion, immune response mediated by T cells (WO2017203529A1).

Crosslinking of hyaluronic acid derivatives is usually performed under alkaline or acidic aqueous media, due to requirements of the crosslinking-agents. Under these conditions (both, acid and alkaline), the vehiculized phytocannabinoids are degraded. In this regard epichlorohydrin, divinylsulfone, 1,4-bisglycidoxybutane, 1,4-butanediol diglucidyl ether (BDDE), 1,2-bis (2,3-epoxypropoxy)ethylene and 1-(2,3-epoxypropyl)-2,3-epoxyciclohexane display an oxirane ring which demands acidic (H+) or alkaline (OH-) aqueous conditions to accomplish the cross-linking (S. Khunmanee, Y. Jeong, H. Park, J. Tissue Eng. 2017, 8, 1-16, doi.org/10.1177/2041731417726464). Similarly, aldehydes such as formaldehyde, glutaraldehyde, crotonaldehyde, taken by themselves or in a mixture require acidic conditions to cross-link the hyaluronic acid (K. Tomihata, Y. Ikada, J Polym Sci A: Polym Chem 1997, 35: 3553-3559). Under alkaline aqueous conditions, water vehiculized phytocannabinoids, cannabidiol and cannabigerol, undergo chemical rearrangement and convert to hydroxyquinones (R. Mechoulan, Z. Ben-Zvi, Tetrahedron 1968, 24, 5615-5624) while under acidic aqueous media CBD rearrange to delta-9-tetrahidrocannabinol *(THC) (*R. Mechoulam, L. Hanus, Chemistry and Physics of Lipids 2002, 121, 35-43) *and CBG ciclyze the terpenyl moiety* Nat. Prod. Rep., 2016, 33, 1357-1392). Therefore, the degradation of phytocannabinoids in acidic or alkaline crosslinking conditions prevents them from fulfilling their function, or their specific applications.

Therefore, a method for the cross-linking of polymers in the presence of vehiculized phytocannabinoids, such as cannabidiol and cannabigerol, that avoid phytocannabinoids degradation, is required.

The method of the present invention allows the cross-linking of hyaluronic acid containing encapsulated or vehiculized phytocannabinoids at neutral pH, avoiding the degradation of the phytocannabinoids.

The effort of the authors of the present invention to perform the operation of crosslinking polymers in the presence of water vehiculized phytocannabinoids, for example cannabidiol (CBD) or cannabigerol (CBG), has led to obtain injectable monophase hydrogels that allow the release of the phytocannabinoids to carry out relevant pharmaceutical, cosmetic and nutraceutical applications, such as the treatment of inflammatory joint diseases and tissue filler applications, taking advance of the viscosupplementation effect of the hyaluronic acid and the anti-inflammatory properties of hyaluronic acid and phytocannabinoids.

### Brief description of the drawings

**Figure 1****.** Controlled release curve of phytocannabinoids. Concentration of CBD (mg/ml) vs time (h) for example 1 composition
**Figure 2****.** Controlled release curve of phytocannabinoids. Concentration of CBG (mg/ml) vs time (h) for example 1 composition.
**Figure 3****.** Controlled release curve of phytocannabinoids. Concentration of CBG+CBD (mg/ml) vs time (h) for example 5 composition.
**Figure 4****.** Controlled release curve of phytocannabinoids. Concentration of CBG+CBD (mg/ml) vs time (h) for example 7 composition.

### Detailed description of the invention

In response to the needs of the state of the art, the authors of the invention have performed new methods for crosslinking polymers in the presence of phytocannabinoids vehiculized through non ionic surfactants. The subsequent product is sterilized, and it is suitable for pharmaceutical applications, for instance for the treatment of inflammatory joint diseases and for tissue filler applications. Non-sterilized product is suitable for cosmetic applications such as moisturizing gels, as well as for nutraceutical applications such as edible gels.

The obtained product is a multimatrix controlled release system of phytocannabinoids based on a cross-linked polymer net (matrix 1) which contains vehiculized phytocannabinoids through non-ionic surfactants (matrix 2). The fabrication of the whole system involves a chemical homogeneous synthesis.

Therefore, in a first aspect, the present invention refers to a controlled release system of phytocannabinoids formulation soluble in aqueous media comprising:
- a phytocannabinoid, or a derivative thereof, or a mixture of phytocannabinoids, or derivatives thereof,
- a non-ionic surfactant, and
- a cross-linked polymer,
wherein the pH of the compositions ranges between 4 and 9, preferably between 6 and 8, and wherein the weight ratio of phytocannabinoid/hyaluronic acid is 50:1 to 1:50, preferably 20:1 to 1:20, more preferably 10:1 to 1:10, and most preferably 2:1 to 1:2, and the weight ratio phytocannabinoid/non-ionic surfactant is 1:30 to 1:1, preferably 1:9 to 1:1. Phytocannabinoids can be obtained not only from natural sources but also from chemical synthesis, biochemical synthesis or from genetically modified microorganism (R. K. Razdan, in The Total Synthesis of Natural Products, ed. J. ApSimon, 1981, vol. 4, pp. 185-262*;* US9822384B2*: Production of cannabinoids in yeast*). Accordingly, phytocannabinoids used in the formulation of the present invention can be natural or synthetic.

Phytocannabinoids can be selected, among others, from cannabigerolic acid, cannabigerolic acid monomethylether, cannabigerol, cannabigerol monomethylether, cannabigerovarinic acid, cannabigerovarin, cannabichromenic acid, cannabichromene, cannabichromevarinic acid, cannabichromevarin, cannabidiolic acid, cannabidiol, cannabidiol monomethylether, cannabidiol C4, cannabidivarinic acid, cannabidivarin, cannabidioreol, D9-(trans)-tetrahydrocannabinolic acid A, delta9-(trans)-tetrahydrocannabinolic acid B, D9-(trans)-tetrahydrocannabinol, D9-(trans)-tetrahydrocannabinolic acid C4, D9-(trans)-tetrahydrocannabinol-C4, D9-(trans)-tetrahydrocannabivarinic acid, D9-(trans)-tetrahydrocannabivarin, D9-(trans)-tetrahydrocannabiorcolic acid, D9-(trans)-tetrahydrocannabiorcol, D8-(trans)-tetrahydrocannabinolic acid, D8-(trans)-tetrahydrocannabinol, cannabicyclolic acid, cannabicyclol, cannabicyclovarin, cannabielsoic acid A, cannabielsoic acid B, cannabielsoin, cannabinolic acid, cannabinol, cannabinol methylether, cannabinol-C4, cannabivarin, cannabiorcol, cannabinodiol, cannabinodivarin, (-)-cannabitriol, (+)-cannabitriol, (±)-9,10-dihydroxy-D 6a(10a)-tetrahydrocannablnol, (-)-10-ethoxy-9-dihydroxy-D 6a(10a)-tetrahydrocannabinol, (±)-8,9-dihydroxy-D 6a(10a)-tetrahydrocannabinol,cannabidiolic acid tetrahydrocannabitriol ester and mixtures thereof.

In a particular embodiment, the phytocannabinoids are selected from cannabidiol, cannabigerol or a mixture thereof. In another particular embodiment, a cannabis sativa extract can be used as phytocannabinoids source. The cannabis sativa extract comes from any part of the cannabis sativa plant including flower, leaf, stem and seeds.

Phytocannabinoids are vehiculized in a non ionic surfactant. In order to aid the vehiculization of the phytocannabinoides, in a particular embodiment, non-ionic triblock copolymers derived of poly(ethylene glycol)-block-poly(propylene glycol)-block-poly(ethyleneglycol) are used as surfactans. The poly(ethylene glycol)-block-poly(propylene glycol)-block-poly(ethyleneglycol) are defined according to the formula: 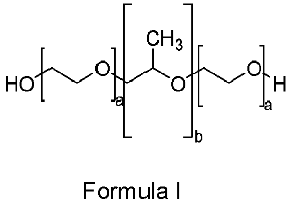 where a is an integer of from 10 to 150 and b is an integer of from 15 to 65.

In a particular embodiment, the formulation may comprise two poly(ethylene glycol)a-block-poly(propylene glycol)b-block-poly(ethyleneglycol)a derivatives. When the formulation comprises two poly(ethylene glycol)a-block-poly(propylene glycol)b-block-poly(ethyleneglycol)a derivatives, it is preferred that for one derivative a is 80, b is 27 and for the other derivative a is 141 and b is 44. Other known poly(ethylene glycol)-block-poly(propylene glycol)-block-poly(ethyleneglycol) derivatives useful in the present invention are those where a is 64 and b is 34, a is 12 and b is 20.

In some particular embodiments, the combination of glyceryl citrate/lactate/linoleate/oleate and polyglyceryl-2 oleate can be used instead of the non-ionic triblock copolymers derived of poly(ethylene glycol)-block-poly(propylene glycol)-block-poly(ethyleneglycol).

The polymer used in the formulations of the present invention can be natural or synthetic polymers. Examples of natural polymers are chondroitin sulfates, keratin sulfate, heparin and heparan sulfate, alginic acid and its biologically acceptable salts, starch, amylose, dextran, xanthan, pullulan, etc. Examples of synthetic polysaccharides are carboxy cellulose, carboxymethyl cellulose, alkyl celluloses such as hydroxyethyl cellulose and hydroxypropyl methyl cellulose (HPMC), oxidized starch etc.

In a preferred embodiment, the polymer is selected from chondroitin sulfate, alginic acid, or a derivative thereof, xanthan gum, carboxy cellulose, carboxymethyl cellulose sodium, hyaluronic acid or a derivative thereof. More preferably, the polymer to be crosslinked is a hyaluronic acid salt. In particular, it is selected from the sodium salt, the potassium salt and mixtures thereof.

In a preferred embodiment the hyaluronic acid salt is of low molecular weight (M), where M ≤0.75·10⁶ Da, or a hyaluronic acid salt of high molecular weight (M), where M ≤2.2·10⁶ Da, or a mixture thereof, more preferably the hyaluronic acid salt is of low molecular weight, where 0.5·10⁶ Da≤ M ≤0.75·10⁶ Da or a hyaluronic acid salt of high molecular weight (M), where 1.9·10⁶ ≤ M ≤2.2·10⁶ Da or a mixture thereof. Said low molecular or high molecular weight salts are of the same nature. In a most preferred embodiment, these salts consist of sodium hyaluronate.

The authors of the invention have performed new methods for obtaining the formulations of the invention, by crosslinking polymers in the presence of phytocannabinoids vehiculized through non ionic surfactants.

Therefore, in a second aspect, the present invention refers to a method of producing the controlled release system of phytocannabinoids formulation soluble in aqueous media comprising the steps of:
a) Obtaining a solution by mixing a polymer, previously dissolved in an aqueous solution, and a non-ionic surfactant,
b) Obtaining a solution of a phytocannabinoid, or a derivative thereof, or a mixture of phytocannabinoids, or derivatives thereof, by dissolving said phytocannabinoids in a proper solvent,
c) Adding the solution obtained in b) to the solution obtained in a), and
d) Cross-linking the resulting solution obtained in c) in the presence of a cross-linking agent.

In a particular embodiment, in step a), a solution that contains a polymer, preferably sodium hyaluronate or a derivative thereof, and a non-ionic surfactant, preferably poly(ethylene glycol)-block-poly(propylene glycol)-block-poly(ethyleneglycol) derivative, is obtained. Upon addition of phytocannabinoids in step b), the non-ionic surfactant is able to form colloidal particles that contain phytocannabinoids.

Solution of step a) comprises low molecular weight and/or high molecular weight sodium hyaluronate in a concentration between 0.1 and 3% (w/w), and preferably in a concentration between 0.5% and 2.5% (w/w) and most preferably in a concentration between 0.75 and 1.5% (w/w), which is a sufficient amount of sodium hyaluronate to guarantee that the crosslinked hyaluronic acid containing phytocannabinoids has a homogeneous consistency.

The aqueous solution medium used in step a) is selected from:
- water,
- a buffer consisting of NaCl, in a concentration between 0.2 to 8%, and Na₂HPO₄ 12H₂O, in a concentration between 0.01% to 10% and NaH₂PO₄ 2H₂O, in a concentration between 0.001% and 10%,
- a buffer consisting of sodium citrate dehydrate or a sodium citrate derivative, in a concentration between 0.002 to 2.5%, and citric acid (hydrated or dehydrated), in a concentration between 0.002% and 2%, or
- a buffer consisting of acetic acid in a concentration between 0.0005% and 0.1% and sodium acetate derivative in a concentration between 0.005% and 0.5%.

In a preferred embodiment, the aqueous solution is a buffer consisting of 1.6% of NaCl, 0.12% of Na₂HPO₄12H₂O and 0.01% of NaH₂PO₄ 2H₂O.

In step b), a phytocannabinoid or a derivative thereof or a mixture of phytocannabinoids or derivatives thereof are dissolved in a proper solvent and added to the solution of step a). Preferably, the phytocannabinoids are selected from cannabidiol, cannabigerol or a mixture thereof. Under these conditions colloidal particles that contain cannabinoids are formed.

In step c), the cross-linking of the resulting solution is carried out in the presence of a cross-linking agent derived from a carbodiimide derivative, a carbonyl imidazole derivative, a carbonyl benzotriazole derivative, a carbonyl triazole derivative or mixtures thereof. Optionally, an active ester forming molecule can be also added to the solution at the cross linking step, for example, N-hydroxysuccinimide (NHS), sulfo-N-hydroxysuccinimide (sulfo-NHS), hydroxybenzotriazole (HOBt), hexafluorophosphate benzotriazole tetramethyl uronium (HBTU) or 1-[bis(dimethylamino)methylene]-1H-1,2,3-triazolo[4,5-b]pyridinium 3-oxide hexafluorophosphate, also called hexafluorophosphate azabenzotriazole tetramethyl uranium (HATU). Preferably, sulfo-NHS is used.

A dihydrazide derivative can also be added in the cross-linking step, for example, one selected from adipic acid dihydrazide, pimelic acid dihydrazide, malonic acid dihydrazide, ethylmalonic acid dihydrazide, carbonyl dihydrazide, oxalyldihydrazide or succinic dihydrazide.

The polymer to be crosslinked is preferably hyaluronic acid salt. It is more preferably selected from the sodium salt, the potassium salt and mixtures thereof, most preferably consists of the sodium salt (NaHA).

In a particular embodiment, the crosslinking process of the method of the invention is a process for crosslinking sodium hyaluronate and derivatives thereof in a solution that contains phytocannabinoids vehiculized in poly(ethylene glycol)-block-poly(propylene glycol)-block-poly(ethyleneglycol) derivatives. The crosslinking process can be applied not only to sodium hyaluronate and derivatives thereof but also to other natural or synthetic polymers. Examples of natural polymers are chondroitin sulfates, keratin sulfate, heparin and heparan sulfate, alginic acid and its biologically acceptable salts, starch, amylose, dextran, xanthan, pullulan, etc. Examples of synthetic polysaccharides are carboxy cellulose, carboxymethyl cellulose, alkyl celluloses such as hydroxyethyl cellulose and hydroxypropyl methyl cellulose (HPMC), oxidized starch etc.

In the context of the cross-linking of this type of polymer (hyaluronic acid salt(s)) and of phytocannabinoids or mixtures thereof, in a preferred embodiment, the cross-linking reaction mixture contains: One hyaluronic acid salt of low molecular weight M, where M ≤0.75·10⁶ Da, preferably 0.5·10⁶ Da≤ M ≤0.75·10⁶ Da or one hyaluronic acid salt of high molecular weight M, where M ≤2.2·10⁶ Da, preferably 1.9·10⁶ ≤ M ≤2.2·10⁶ Da, or a mixture thereof. Said low molecular or high molecular weight salts are of the same nature and very advantageously consisting of sodium hyaluronate.

Sometimes, during the cross-linking process, the pH of the formulation may be outside the desired ranges (6 and 8), and then, a pH adjusting step after the cross-linking is necessary, wherein:
- If pH reached at the crosslinking is too acidic, the pH adjusting step proceeds by addition of a 0.25 M solution of sodium hydroxide (NaOH) until the required pH is reached, or
- If pH reached in step d) is basic, the pH adjusting step proceeds by addition of a 0.25 M solution of chlorhydric acid (HCI) until the required pH is reached.

In a particular embodiment, the solvent used to dissolve the phytcannabinoid in any of the method of the invention, is defined by the formula: 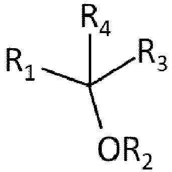 wherein R1-R4 are selected independently from H, OH, CH₂OH, CH₃, CH₂CH₃, C(O)CH₃, C(O)OCH₂CH₃, CH₂C(O)CH₂CH₃.

The solvent is preferably selected from the group consisting of methanol, 1-propanol and isomers thereof, ethylene glycol, propylene glycol and mixtures thereof.

The crosslinking step involves the addition of a carbodiimide derivative. In a particular embodiment, the carbodiimide derivative is defined by the formula:

R¹-N=C=N-R²

wherein R1 can be equal to R2. R1 and R2 are selected from cyclohexyl, isopropyl, 3-dimethylaminopropyl, ethyl, (2-morpholinoethyl). The carbodiimide can be as well in form hydrochloride or in form of methoxy-p-toluenesulfonate.

Instead of a carbodiimide derivative another crosslinking agents such as 1,1'-carbonyl diimidazole carbonyldibenzimidazole, carbonyldi-1,2,4-triazole, and carbonyldibenzotriazole may be used.

Crosslinking process could require the addition of an active ester forming molecule. Example of active ester forming molecule are N-hydroxysuccinimide (NHS), sulfo-N-hydroxysuccinimide (sulfo-NHS), hydroxybenzotriazole (HOBt), hexafluorophosphate benzotriazole tetramethyl uronium (HBTU), 1-[bis(dimethylamino)methylene]-1H-1,2,3-triazolo[4,5-b]pyridinium 3-oxide hexafluorophosphate also called hexafluorophosphate azabenzotriazole tetramethyl uranium (HATU). In a preferred embodiment sulfo-N-hydroxysuccinimide (sulfo-NHS) is used.

Cross-linking is also performed by the addition of dihydrazide derivatives to the crosslinking mixture that contains carbodiimide derivatives as well as active ester forming molecules. Examples of dihydrazide derivatives are adipic acid dihydrazide, pimelic acid dihydrazide, malonic acid dihydrazide, ethylmalonic acid dihydrazide, carbonyl dihydrazide, oxalyldihydrazide, succinic dihydrazide.

Alternatively, the present invention contemplates another method of producing the controlled release system of phytocannabinoids formulation soluble in aqueous of the present invention comprising the steps of:
i. Obtaining a solution by dissolving a polymer in an aqueous solution,
ii. Obtaining a solution of a phytocannabinoid, or a derivative thereof, or a mixture of phytocannabinoids, or derivatives thereof, by dissolving said phytocannabinoids in a solution that contains a non-ionic surfactant and a proper solvent,
iii. Adding the solution obtained in ii) to the solution obtained in i), and
iv. Cross-linking the resulting solution obtained in iii) in the presence of a cross-linking agent.

Solution of step i) contains low molecular weight and/or high molecular weight sodium hyaluronate in a concentration between 0.1 and 3%, and preferably in a concentration between 0.5% and 2.5% and most preferably in a concentration between 0.75 and 1.5%, which is a sufficient amount of sodium hyaluronate to guarantee that the crosslinked hyaluronic acid containing phytocannabinoids has a homogeneous consistency.

The aqueous solution medium used in step i) is selected from:
- water,
- a buffer consisting of NaCl, in a concentration between 0.2 to 8%, and Na₂HPO₄ 12H₂O, in a concentration between 0.01% to 10% and NaH₂PO₄ 2H₂O, in a concentration between 0.001% and 10%,
- a buffer consisting of sodium citrate dehydrate or a sodium citrate derivative, in a concentration between 0.002 to 2.5%, and citric acid (hydrated or dehydrated), in a concentration between 0.002% and 2%, or
- a buffer consisting of acetic acid in a concentration between 0.0005% and 0.1% and sodium acetate derivative in a concentration between 0.005% and 0.5%.

In a preferred embodiment, the aqueous solution is a buffer consisting of 1.6% of NaCl, 0.12% of Na₂HPO₄12H₂O and 0.01% of NaH₂PO₄ 2H₂O.

In a particular embodiment, in step ii) a cannabis sativa extract containing phytocannabinoids is dissolved in a solution that contains a combination of glyceryl citrate/lactate/linoleate/oleate and polyglyceryl-2 oleate, as non-ionic surfactant, and a proper solvent, preferably propylenglycol. Under these conditions, nanocapsules which contain cannabinoids are formed.

Sometimes, during the cross-linking process, the pH of the formulation may be outside the desired ranges (6 and 8), and then, a pH adjusting step after the cross-linking is necessary, wherein
- If pH reached at the crosslinking is too acidic, the pH adjusting step proceeds by addition of a 0.25 M solution of sodium hydroxide (NaOH) until the required pH is reached, or
- If pH reached in step d) is basic, the pH adjusting step proceeds by addition of a 0.25 M solution of chlorhydric acid (HCI) until the required pH is reached.

In a particular embodiment, the solvent used to dissolve the phytcannabinoid in any of the method of the invention, is defined by the formula: 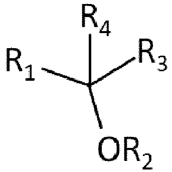 wherein R1-R4 are selected independently from H, OH, CH₂OH, CH₃, CH₂CH₃, C(O)CH₃, C(O)OCH₂CH₃, CH₂C(O)CH₂CH₃.

The crosslinking step involves the addition of a carbodiimide derivative. In a particular embodiment, the carbodiimide derivative is defined by the formula:

R¹-N=C=N-R²

wherein R1 can be equal to R2. R1 and R2 are selected from cyclohexyl, isopropyl, 3-dimethylaminopropyl, ethyl, (2-morpholinoethyl). The carbodiimide can be as well in form hydrochloride or in form of methoxy-p-toluenesulfonate.

Instead of a carbodiimide derivative another crosslinking agents such as 1,1'-carbonyl diimidazole carbonyldibenzimidazole, carbonyldi-1,2,4-triazole, and carbonyldibenzotriazole may be used.

Crosslinking process could require the addition of an active ester forming molecule. Example of active ester forming molecule are N-hydroxysuccinimide (NHS), sulfo-N-hydroxysuccinimide (sulfo-NHS), hydroxybenzotriazole (HOBt), hexafluorophosphate benzotriazole tetramethyl uronium (HBTU), 1-[bis(dimethylamino)methylene]-1H-1,2,3-triazolo[4,5-b]pyridinium 3-oxide hexafluorophosphate also called hexafluorophosphate azabenzotriazole tetramethyl uranium (HATU). In a preferred embodiment sulfo-N-hydroxysuccinimide (sulfo-NHS) is used.

Cross-linking is also performed by the addition of dihydrazide derivatives to the crosslinking mixture that contains carbodiimide derivatives as well as active ester forming molecules. Examples of dihydrazide derivatives are adipic acid dihydrazide, pimelic acid dihydrazide, malonic acid dihydrazide, ethylmalonic acid dihydrazide, carbonyl dihydrazide, oxalyldihydrazide, succinic dihydrazide.

The resulting formulation, according to the methods of the invention, exhibit a concentration of hyaluronic acid between 0.1 and 3% (w/w), and preferably in a concentration between 0.5% and 2.5% (w/w) and most preferably in a concentration between 0.75 and 1.5% (w/w) and of cross-linking reagents in a concentration between 0.000025 M and 0.5 M and preferably between 0.05 M and 0.3 M and most preferably between 0.1 and 0.2 M. In a particular embodiment, for N-(3-dimethylaminopropyl)-N'-ethylcarbodiimide hydrochloride the concentrations in weight are between 0.0004% and 8% (w/w), and preferably between 0.8% and 5% (w/w), and most preferably between 1.6 and 3.2% (w/w) with a pH between 4.5 and 8, which are compatible with an injectable use.

After the crosslinking step, the resulting formulation can be optionally sterilized, as in the case of injectable formulations. Sterilization process may be performed by steam sterilization, in an autoclave at a temperature ranging from 120°C to 140°C. In particular, the sterilization can be performed at 121° for 15 to 20 minutes, preferably 15 min, to obtain F0>15 (sterilizing value). Dry-heat is also employed to achieve sterilization.

Sterilization may be performed by other means such as radiation sterilization including UV, X-rays, gamma ray, beta particles (electrons).

Sterilization may be also realized by chemical means including ethylene oxide, carbon-dioxide, ozone gas, hydrogen peroxide, nitrogen dioxide, glutaraldehyde and formaldehyde solutions, phthalaldehyde and peracetic acid.

The methods of the present invention afford scalability so that the fabrication method can be performed at industrial level (manufacturing). The method provides a net that includes homogeneously distributed colloid particles or nanocapsules and afford a controlled release system which can be optimized by:
- Hyaluronic acid with different molecular weight,
- Ratio of hyaluronic acid molecules with different molecular weight,
- Ratio hyaluronic acid /water,
- Cross-linking degree indicated by the cross-linking percentage,
- Cross-linking agent,
- % of colloid particles and nanocapsules,
- % of phytocannabinoids,
- Loading of colloid particles with different concentration (%) of phytocannabinoids,
- Type of phytocannabinoid and combination of them,
- Modulation of controlled release curves of phytocannabinoids, and
- Terminal sterilization.

The compositions of the invention are suitable for pharmaceutical, cosmetic and nutraceutical applications.

Therefore, in another aspect, the invention refers to a pharmaceutical composition comprising the phytocannabinoid formulations of the present invention and their uses in different pharmaceutical or medical applications. In particular, the present invention refers to the use of this pharmaceutical composition in the treatment of inflammatory joint diseases, taking advance of the viscosupplementation effect of the hyaluronic acid and the anti-inflammatory properties of hyaluronic acid and cannabinoids, especially cannabidiol.

The invention also refers to a pharmaceutical composition comprising the phytocannabinoid formulations of the present invention and its use in tissue filler applications. Specifically, the pharmaceutical composition of the invention affords sterile soft tissue filler compositions for the augmentation and/or repair of soft tissue and keratin materials, like the skin. These compositions can also comprise local anesthetics, such as lidocaine.

The present invention also refers to a cosmetic composition comprising the controlled release system of the present invention and its use for cosmetic applications. Specifically, the cosmetic composition of the invention affords compositions with relaxing, soothing and moisturizing effects on the skin.

Finally, the composition of the invention also refers to a nutraceutical composition comprising the controlled release system of the present invention and its use for nutraceutical applications. Specifically, nutraceutical compositions of the invention are useful for relaxation, calming and moisturization of the skin and for the improvement of the well-being of the human body.

The compositions of the invention can be administered by topical administration. Suitable topical compositions can be gels, ointments, creams, lotions, drops, etc. Topical compositions obtained by the methods of the invention do not need a sterilization step after the crosslinking step.

The composition of the invention can also be administered by systemic administration. This includes delivering the phytocannabinoid composition by injection, wherein the injection is intravenous, intra-articular, intramuscular, intradermal, intraspinal, intraperitoneal, subcutaneous, a bolus or a continuous administration.

The composition of the invention can also be administered by oral administration, such edible gels in the case of nutraceutical compositions.

The compositions of the invention can be administered including in a medical device. In an example, the composition can be drawn into a syringe for a water-based injection medium.

### Examples

### Analytical Techniques:

### Reometry

The consistency of the gel is characterized at 25°C by rheological measurement of the moduli of elasticity (G') and viscosity (G") as a function of the frequency (from 10 Hz to 0.01 Hz) using a controlled strain (1%) in AR 550 Rheometer (TA Instruments) and a cone-and-plate geometry of 40 mm diameter and a truncation (gap) of 115 µm.

### High Performance Liquid Cromatography (HPLC)

This technique was used to determine the total content of phytocannabinoids (CBG/CBD) in the formulations and the amount of CBG/CBD encapsulated in the colloidal particles.

The analysis of the total amount of CBG/CBD in the system was performed by direct dilution of the samples in methanol followed by filtration through disposable 0.22 µm PVDF filters and subsequent injection in the chromatography equipment.

A HPLC-DAD analytical method according to **Table 1** was developed in order to quantify the CBG and CBD concentration in the formulations. Such a method is common for both CBG and CBD.

**Table 1 Chromatographic method for the quantification of CBG and CBD.**

| | |
|---|---|
| System | HPLC (1260 series Agilent Technologies) |
| Column | Zorbax Eclipse XCB-C18 (150×4.6 mm, 5 µm particle, Agilent) |
| Mobile-Phase | Channel A: Ammonium formate 10 mM (pH 3.6, with formic acid) |
| | Channel B: Acetonitrile |
| Gradient | 0-4 min 52-80% of B; 4-9.5 min 80 % of B at 1 ml/min (post-run= 2 min) |
| Detector | DAD (210, 228 and 270 nm) |

In this method a stock solution of 1000 mg/L of CBG/CBD in ethanol was prepared from which 1, 2, 3, 4, 5 and 6 mg/L standard dilutions of CBG/CBD in ethanol were made.

### Dynamic Light Scattering (DLS)

DLS measurements were performed by diluting 70 µL of the samples in 900 µL of water followed by analysis in the DLS equipment at 173° measurement angle. Atenuator value, measurement position and count number were employed as measurement quality indicators (table 2).

**Table 2: DLS analysis method parameters for the characterization of vehiculized CBG.**

| Dispersant | Water |
|---|---|
| Temperature | 25°C |
| Viscosity | 0.8872 cP |
| RI | 1.330 |
| Dielectric constant | 78.5 |
| Fitting model | Smoluchowski |
| Equilibration time | 120 s |
| Cell time | Capillary cell DTS1070 |
| Measurement angle | 173° |
| Number of measurements per sample | 3 |

### EXAMPLE 1.

1 g of low molecular weight hyaluronic acid (MW 0.5-0.75 MDa) was dissolved in 99 mL phosphate saline buffer (pH 7.4) for 12 hours. 0.270 g of poly(ethylene glycol)a- - poly(propylene glycol)*b*-block-poly(ethyleneglycol)*a* where *a*=80, *b*=27 and 0.270 g poly(ethylene glycol)*a*-block-poly(propylene glycol)*b*-block-poly(ethyleneglycol)*a* where *a*=141 *b*=44 was added via spatula to 5.40 g of hyaluronic acid solution. The resulting solution was stirred mechanically until complete solution of the polymers was reached. Following a solution of phytocannabinoid (64 mg of CBD, or 64 mg of CBG or a mixture of 32 mg of CBG + 32 mg of CBD) in 100 microL of an organic solvent (methanol, isopropanol or propyleneglycol) was added to the hyaluronic acid/poly(ethylene glycol)*a*-poly(propylene glycol)*b*-block-poly(ethyleneglycol)*a* solution. The resulting solution was stirred mechanically for 48 hours. To that solution 230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically until the complete dissolution of the reactants was reached.

### EXAMPLE 2.

The experiment of example 1 was repeated adding a new component: adipic dihydrazide to the cross-linking step. The protocol was modified as follows:
230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically for 1 hour. Afterwards 262 mg of adipic dihydrazide were added and the resulting mixture was shaken until complete dissolution of the dihydrazide is reached. The resulting mixture was let to cross-link for 24 h.

### EXAMPLE 3

The experiment of example 1 was modified so that half amount of EDC*HCl and of sulfo-NHS were used for the cross-linking step. The protocol was adapted as follows:
115 mg of N-(3-dimethylaminopropyl)-N'-ethylcarbodiimide hydrochloride (EDC*HCl) and 33 mg of N-hydroxysulfosuccinimide sodium salt (sulfo-NHS) was added via spatula. The resulting mixture was shaken mechanically until the complete dissolution of the reactants was reached.

### EXAMPLE 4

0.9 g of low molecular weight hyaluronic acid (MW 0.5 - 0.75 ·10⁶ Da) and 0.1 g of high molecular weight hyaluronic acid (MW 1.9 - 2.2 ·10⁶ Da) were dissolved in 99 mL phosphate saline buffer (pH 7.4) for 12 hours. 0.270 g of poly(ethylene glycol)*a*-block-poly(propylene glycol)*b*-block-poly(ethyleneglycol)*a* where *a*=80, *b*=27 and 0.270 g poly(ethylene glycol)*a-*block-poly(propylene glycol)*b*-block-poly(ethyleneglycol)*a* where *a*=141 *b*=44 was added via spatula to 5.40 g of hyaluronic acid solution. The resulting solution was stirred mechanically until complete solution of the polymers was reached. Following a solution of phytocannabinoid (96 mg of CBD, or 96 mg of CBG or a mixture of 48 mg of CBG + 48 mg of CBD) in 150 microL of an organic solvent (methanol, isopropanol or propyleneglycol) was added to the hyaluronic acid/poly(ethylene glycol)*a*-poly(propylene glycol)*b*-block-poly(ethyleneglycol)a solution. The resulting solution was stirred mechanically for 48 hours. To that solution 230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically until the complete dissolution of the reactants was reached.

### EXAMPLE 5

The experiment of example 4 was modified so that half amount of EDC*HCl and of sulfo-NHS were used for the cross-linking step. The protocol was adapted as follows:
115 mg of N-(3-dimethylaminopropyl)-N'-ethylcarbodiimide hydrochloride (EDC*HCl) and 33 mg of N-hydroxysulfosuccinimide sodium salt (sulfo-NHS) was added via spatula. The resulting mixture was shaken mechanically until the complete dissolution of the reactants was reached.

### EXAMPLE 6

0.5 g of low molecular weight hyaluronic acid (MW 0.5 - 0.75 ·10⁶ Da) and 0.5 g of high molecular weight hyaluronic acid (MW 1.9 - 2.2 ·10⁶ Da) were dissolved in 99 mL phosphate saline buffer (pH 7.4) for 12 hours. 0.270 g of poly(ethylene glycol)a-block-poly(propylene glycol)*b*-block-poly(ethyleneglycol)*a* where *a*=80, *b*=27 and 0.270 g poly(ethylene glycol)*a-*block-poly(propylene glycol)*b*-block-poly(ethyleneglycol)*a* where *a*=141 *b*=44 was added via spatula to 5.40 g of hyaluronic acid solution. The resulting solution was stirred mechanically until complete solution of the polymers was reached. Following a solution of phytocannabinoid (96 mg of CBD, or 96 mg of CBG or a mixture of 48 mg of CBG + 48 mg of CBD) in 150 microL of an organic solvent (methanol, isopropanol or propyleneglycol) was added to the hyaluronic acid/poly(ethylene glycol)a-poly(propylene glycol)*b*-block-poly(ethyleneglycol)a solution. The resulting solution was stirred mechanically for 48 hours. To that solution 230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically until the complete dissolution of the reactants was reached.

### EXAMPLE 7

The experiment of example 6 was modified so that half amount of EDC*HCl and of sulfo-NHS were used for the cross-linking step. The protocol was adapted as follows:
115 mg of N-(3-dimethylaminopropyl)-N'-ethylcarbodiimide hydrochloride (EDC*HCl) and 33 mg of N-hydroxysulfosuccinimide sodium salt (sulfo-NHS) was added via spatula. The resulting mixture was shaken mechanically until the complete dissolution of the reactants was reached.

### EXAMPLE 8

1g of high molecular weight hyaluronic acid (MW 1.9 - 2.2 ·10⁶ Da) were dissolved in 99 mL phosphate saline buffer (pH 7.4) for 12 hours. 0.270 g of poly(ethylene glycol)a-block-poly(propylene glycol)b-block-poly(ethyleneglycol)a where a=80, b=27 and 0.270 g poly(ethylene glycol)a-block-poly(propylene glycol)b-block-poly(ethyleneglycol)a where a=141 b=44 was added via spatula to 5.40 g of hyaluronic acid solution. The resulting solution was stirred mechanically until complete solution of the polymers was reached. Following a solution of phytocannabinoid (64 mg of CBD, or 64 mg of CBG or a mixture of 32 mg of CBG + 32 mg of CBD) in 100 microL of an organic solvent (methanol, isopropanol or propyleneglycol) was added to the hyaluronic acid/poly(ethylene glycol)*a*-poly(propylene glycol)*b*-block-poly(ethyleneglycol)*a* solution. The resulting solution was stirred mechanically for 48 hours. To that solution 230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically until the complete dissolution of the reactants was reached.

### EXAMPLE 9.

1 g of low molecular weight hyaluronic acid (MW 0.5-0.75 MDa) was dissolved in 10 mL phosphate saline buffer (pH 7.4) for 12 hours. Following 0.60 grams of Mc Beauty Science Nano CBD Capsules (5%) were added to 5.4 g of the hyaluronic acid solution. The resulting solution was stirred mechanically for 48 hours. To that solution 230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically until the complete dissolution of the reactants was reached. The resulting mixture was let to cross-link for 24 h.
INCI Name: Water (and) Cannabis Sativa Flower/Leaf/Stem Extract (and) Propylene Glycol (and) Glyceryl Citrate/Lactate/Linoleate/Oleate (and) Polyglyceryl-2 Oleate
CAS No.: 7732-18-5, 57-55-6, 9174-23-9, 9007-48-1
Composition (acc. to FDA):
A: > 50% Water
C: 10-25% Cannabis Sativa Flower/Leaf/Stem Extract
C: 10-25% Propylene Glycol
4.8-5.2% Cannabidiol
E: 1-5% Glyceryl Citrate/Lactate/Linoleate/Oleate
E: 1-5% Polyglyceryl-2 Oleate

### EXAMPLE 10

The experiment of example 9 was repeated adding a new component: adipic dihydrazide to the cross-linking step. The protocol was modified as follows:
230 mg of N-(3-dimethylaminopropyl)-N'-ethylcarbodiimide hydrochloride (EDC*HCl) and 65 mg of N-hydroxysulfosuccinimide sodium salt (sulfo-NHS) was added via spatula. The resulting mixture was shaken mechanically for 1 hour. Afterwards 262 mg of adipic dihydrazide were added and the resulting mixture was shaken until complete dissolution of the dihydrazide is reached. The resulting mixture was let to cross-link for 24 h.

### EXAMPLE 11. Steam sterilization.

1.2 g of material from EXAMPLES 1-9 were loaded manually in a rubber capped injection barrel of a 1.5 mL glass body syringe. The piston rod is plugged into the loaded injection barrel and the resulting loaded syringe is submitted to steam sterilization at 121°C for 15 minutes.

### EXAMPLE 12. Controlled release experiment.

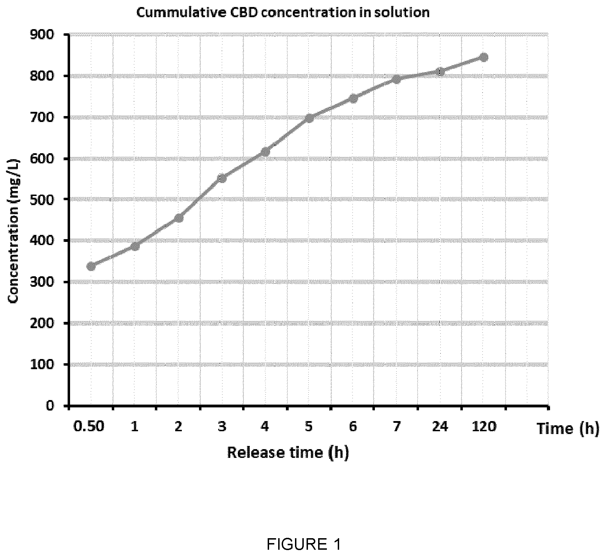

2.59 grams of material containing CBD from example 1 were introduced in a membrane that allows passing of the vehiculized cannabinoid through it. Filled membrane was put into contact with 29.91 g of phosphate buffer saline. The resulting solution was called controlled released solution. At different times 1 mL of solution was removed from the controlled release solution and the content was analysed by HPLC. For every 1 mL of controlled release solution that was removed for HPLC analysis another 1 mL of phosphate buffer saline was added to the controlled release solution so that the volume stayed constant (Fig. 1). Figure 1 shows the controlled release of colloidal particles that contain CBD in a phosphate buffer medium. The analyzed concentration of CBD is cumulative, which means that concentration refers to the actual concentration of CBD in the controlled release solution and reaches a maximum value of 847 mg/L after 120h of release experiment. 95% of the release of CBD takes place in the first 24h of the experiment.

### EXAMPLE 13. Controlled release experiment.

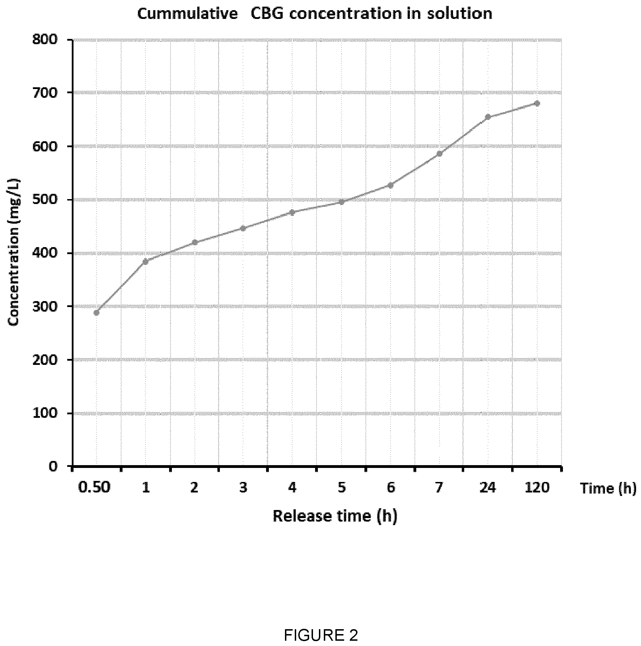

2.45 grams of material containing CBG from example 1 were introduced in a membrane that allows passing of the vehiculized cannabinoid through it. Filled membrane was put into contact with 29.98 g of phosphate buffer saline. The resulting solution was called controlled released solution. At different times 1 mL of solution was removed from the controlled release solution and the content was analysed by HPLC. For every 1 mL of controlled release solution that was removed for HPLC analysis another 1 mL of phosphate buffer saline was added to the controlled release solution so that the volume stayed constant (Fig. 2). Figure 2 shows the controlled release of colloidal particles that contain CBG in a phosphate buffer medium. The analyzed concentration of CBG is cumulative, which means that concentration refers to the actual concentration of CBG in the controlled release solution and reaches a maximum value of 680 mg/L after 120h of release experiment. 96% of the release of CBD takes place in the first 24h of the experiment.

### EXAMPLE 14. Controlled release experiment.

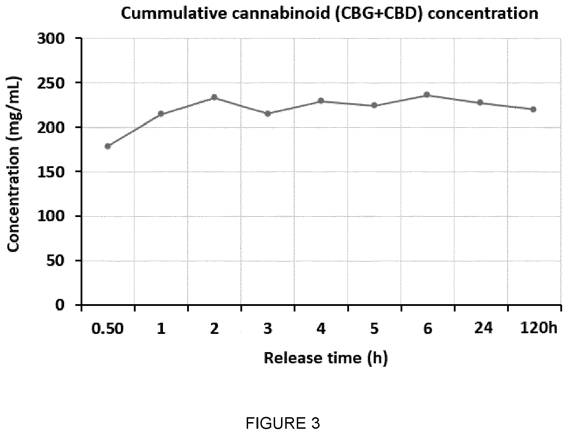

2.43 grams of material containing CBD and CBG from example 5 were introduced in a membrane that allows passing of the vehiculized cannabinoid through it. Filled membrane was put into contact with 28.82 g of phosphate buffer saline. The resulting solution was called controlled released solution. At different times 1 mL of solution was removed from the controlled release solution and the content was analysed by HPLC. For every 1 mL of controlled release solution that was removed for HPLC analysis another 1 mL of phosphate buffer saline was added to the controlled release solution so that the volume stayed constant (Fig. 3). Figure 3 shows the controlled release of colloidal particles that contain a mixture of CBG+CBD in a phosphate buffer medium. The analyzed concentration of cannabinoids is cumulative, which means that concentration refers to the actual concentration of cannabinoids in the controlled release solution and reaches a maximum value of 235 mg/L after 6h of release experiment. 98% of the maximum release of CBD takes place after 2h of the release experiment.

### EXAMPLE 15. Controlled release experiment.

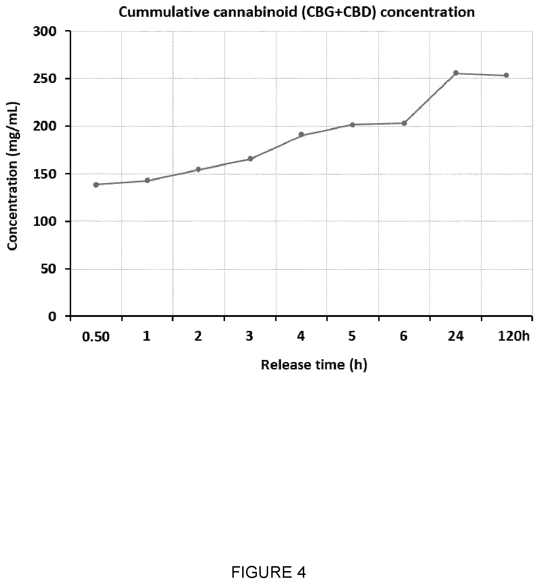

2.68 grams of material containing CBD and CBG from example 7 were introduced in a membrane that allows passing of the vehiculized cannabinoid through it. Filled membrane was put into contact with 29.33 g of phosphate buffer saline. The resulting solution was called controlled released solution. At different times 1 mL of solution was removed from the controlled release solution and the content was analysed by HPLC. For every 1 mL of controlled release solution that was removed for HPLC analysis another 1 mL of phosphate buffer saline was added to the controlled release solution so that the volume stayed constant (Fig. 4). Figure 4 shows the controlled release of colloidal particles that contain a mixture of CBG+CBD in a phosphate buffer medium. The analyzed concentration of cannabinoids is cumulative, which means that concentration refers to the actual concentration of cannabinoids in the controlled release solution and reaches a maximum value of 255 mg/L after 24h of release experiment. 79% of the maximum release of CBD takes place after 6h of the release experiment.

### EXAMPLE 16. Physicochemical and rheological characterization of sterilized formulations

| Composition | Cannabinoid % (HPLC) | Cross-linking | pH | Size of particles (nm) | PDI | Zeta potential (mV) | G' | G" |
|---|---|---|---|---|---|---|---|---|
| 1 | CBG 0.67% | Method example 1 | 7.3 | 46.3 | 0.475 | -33.6 | 28.2 | 5.9 |
| 2 | CBD 0.35% | Method example 1 | 7.3 | 44.5 | 0.667 | -8.0 | 26.9 | 6.0 |
| 3 | CBG 0.39% | Method example 2 | 6.3 | 45.5 | 0.576 | -7.8 | 3.3 | 2.6 |
| 4 | CBD 0.35% | Method example 2 | 6.3 | 57.8 | 0.561 | -15.1 | 45.0 | 5.6 |
| 5 | CBG 0.60% | Method example 3 | 7.2 | 57.5 | 0.346 | -21.3 | 65.6 | 8.6 |
| 6 | CBD 0.41% | Method example 3 | 7.4 | 57.8 | 0.285 | -29.4 | 38.9 | 8.1 |
| 7 | CBD 0.55% | Method example 4 | 6.0 | 47.2 | 0.344 | -45.1 | 31.9 | 12.0 |
| | CBG 0.58% | | | | | | | |
| 8 | CBD 0.58% | Method example 5 | 7.0 | 39.0 | 0.383 | -16.8 | 46.3 | 10.4 |
| | CBG 0.61% | | | | | | | |
| 9 | CBD: 0.38% | Method example 6 | 6.0 | 72.2 | 0.564 | -47.2 | 19.9 | 11.0 |
| | CBG: 0.38% | | | | | | | |
| 10 | CBD: 0.59% | Method example 7 | 7.1 | 60.3 | 0.761 | -18.9 | 50.2 | 12.7 |
| | CBG: 0.56% | | | | | | | |
| 11 | CBG 0.99% | Method example 8 | 7.5 | 309.1 | 0.488 | -18.4 | 77.0 | 13.5 |
| 12 | CBD 0.79% | Method example 8 | 7.5 | 60.8 | 0.297 | -25.1 | 20.5 | 5.7 |
| 13 | CBD 0.17% | Method example 9 | 7.2 | 69.4 | 1.0 | -17.8 | 15.4 | 3.8 |

- Data from example 16 show that: Cannabinoids or mixtures of them are incorporated into colloidal particles based on non-ionic surfactants. Such particles are embedded in a matrix of crosslinked hyaluronic acid derivatives.
- The size of the colloidal particles is well defined and display polydispersity indexes (PDIs) down to 0.285. Such PDI values are typical for populations of particles with homogenous size.
- The zeta potential data with absolute values up to 47 mV indicates that the colloidal particles, which contain cannabinoids, are very stable and with low tendency to aggregation.
- G' (elastic modulus) is larger than G" (viscous modulus) which indicates that the elastic behaviour of the composition predominates over the viscous behaviour and demonstrates that the hyaluronic acid derivatives are chemically crosslinked (Stefano Santoro, Luisa Russo, Vincenzo Argenzio, Assunta Borzacchiello. Rheological properties of cross-linked hyaluronic acid dermal fillers. J. Appl Biomater Biomech 2011; Vol. 9 no. 2, 127-136).
- The use of lower amounts of crosslinking agents afford formulation with higher G' and G" values (example 3 vs example 1; example 5 vs example 4; example 7 vs example 6).

## Claims

1. A controlled release system of phytocannabinoids formulation soluble in aqueous media comprising:
- a phytocannabinoid, or a derivative thereof, or a mixture of phytocannabinoids, or derivatives thereof,
- a non-ionic surfactant,
- a cross-linked polymer,
wherein the pH of the compositions ranges between 4 and 9, preferably between 6 and 8, and wherein the weight ratio of cannabinoid/hyaluronic acid is 50:1 to 1:50, preferably 20:1 to 1:20, and more preferably 10:1 to 1:10, and most preferably 2:1 to 1:2, and the weight ratio cannabinoid/non-ionic surfactant is 1:30 to 1:1, preferably 1:9 to 1:1.

2. A controlled release system according to claim 1 wherein the cannabinoids are phytocannabinoids selected from cannabidiol, cannabigerol or a mixture thereof.

3. A controlled release system according to claim 1 or 2 wherein the non-ionic surfactant is at least one poly (ethylene glycol)-block-poly (propylene glycol)-block-poly(ethyleneglycol) derivative.

4. A controlled release system according to claim 3 wherein the poly (ethylene glycol)-block-poly (propylene glycol)-block-poly(ethyleneglycol) derivative is defined according to the formula: 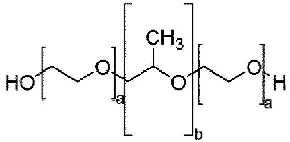 wherein a is an integer of from 10 to 150 and b is an integer of from 15 to 65.

5. A controlled release system according to claim 4 wherein the non-ionic surfactant comprises two poly(ethylene glycol)a-block-poly(propylene glycol)b-block-poly(ethyleneglycol)a derivatives.

6. A controlled release system according to claim 5 wherein for one derivative a is 80 and b is 27 and for the other derivative a is 141 and b is 44.

7. A controlled release system according to claim 5 wherein for one derivative a is 64 and b is 34, and for the other derivative a is 12 and b is 20.

8. A controlled release system according to claim 1 wherein the phytocannabinoid is a cannabis sativa extract.

9. A controlled release system according to claim 8 wherein the non-ionic surfactant is a combination of glyceryl citrate/lactate/linoleate/oleate and polyglyceryl-2 oleate.

10. A controlled release system according to any of previous claims, wherein the polymer is selected from chondroitin sulfate, alginic acid, or a derivative thereof, xanthan gum, carboxy cellulose, carboxymethyl cellulose sodium, hyaluronic acid or a derivative thereof.

11. A controlled release system according to claim 10, wherein the polymer is a hyaluronic acid salt.

12. A controlled release system according to claim 11, wherein the hyaluronic acid salt is of low molecular weight (M), where M ≤50.75·10⁶ Da, or a hyaluronic acid salt of high molecular weight (M), where M ≤2.2·10⁶ Da, or a mixture thereof.

13. A controlled release system according to claim 12 wherein the hyaluronic acid salt is of low molecular weight, where 0.5·10⁶ Da≤ M ≤0.75·10⁶ Da or a hyaluronic acid salt of high molecular weight (M), where 1.9·10⁶ ≤ M ≤2.2·10⁶ Da or a mixture thereof.

14. A controlled release system according to any of claims 11-13 wherein the hyaluronic acid salt is sodium hyaluronate.

15. A method of producing a controlled release system of cannabinoids formulation soluble in aqueous media according to claim 1 comprising the steps of:
a) Obtaining a solution by mixing a polymer, previously dissolved in an aqueous solution, and a non-ionic surfactant,
b) Obtaining a solution of a cannabinoid, or a derivative thereof, or a mixture of cannabinoids, or derivatives thereof, by dissolving said cannabinoids in a proper solvent,
c) Adding the solution obtained in b) to the solution obtained in a), and
d) Cross-linking the resulting solution obtained in c) in the presence of a cross-linking agent.

16. The method according to claim 15 wherein the phytocannabinoids used in step b) are selected from cannabidiol, cannabigerol or a mixture thereof.

17. A method, according to claim 15 or 16, wherein the non-ionic surfactant used in step a) is at least one poly (ethylene glycol)-block-poly (propylene glycol)-block-poly(ethyleneglycol) derivative.

18. Method according to claim 17 wherein the poly (ethylene glycol)-block-poly (propylene glycol)-block-poly(ethyleneglycol) derivative is defined according to the formula: 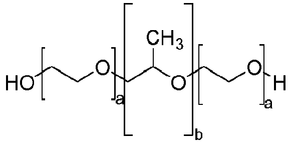 wherein a is an integer of from 10 to 150 and b is an integer of from 15 to 65.

19. The method according to claim 18 wherein the non-ionic surfactant comprises two poly(ethylene glycol)a-block-poly(propylene glycol)b-block-poly(ethyleneglycol)a derivatives.

20. The method according to claim 19 wherein for one derivative a is 80 and b is 27 and for the other derivative a is 141 and b is 44.

21. The method according to claim 19 wherein for one derivative a is 64 and b is 34, and for the other derivative a is 12 and b is 20.

22. A method of producing a controlled release system of phytocannabinoids formulation soluble in aqueous media according to claim 1 comprising the steps of:
i. Obtaining a solution by dissolving a polymer in an aqueous solution,
ii. Obtaining a solution of a phytocannabinoid, or a derivative thereof, or a mixture of phytocannabinoids, or derivatives thereof, by dissolving said phytocannabinoids in a solution that contains a non-ionic surfactant and a proper solvent
iii. Adding the solution obtained in ii) to the solution obtained in i), and
iv.Cross-linking the resulting solution obtained in iii) in the presence of a cross-linking agent.

23. The method according to claim 22 wherein a cannabis sativa extract containing phytocannabinoids is used in step i).

24. The method according to claim 23 wherein the non-ionic surfactant used in step ii) is glyceryl citrate/lactate/linoleate/oleate and polyglyceryl-2 oleate.

25. Method according to any of claims 15-24 further comprising a pH adjusting step after the cross-linking step, wherein
- when pH reached after the crosslinking step is acidic, the pH adjusting step proceeds by addition of a 0.25 M solution of sodium hydroxide (NaOH) until the required pH is reached, or
- when pH reached in step d) is basic, the pH adjusting step proceeds by addition of a 0.25 M solution of chlorhydric acid (HCI) until the required pH is reached.

26. Method according to any of claims 15-25 wherein the polymer is selected from chondroitin sulfate, alginic acid, or a derivative thereof, xanthan gum, carboxy cellulose, carboxymethyl cellulose sodium, hyaluronic acid or a derivative thereof.

27. Method according to claim 26 wherein the polymer is a hyaluronic acid salt of low molecular weight (M), where M ≤0.75·10⁶ Da, or a hyaluronic acid salt of high molecular weight (M), where M ≤2.2·10⁶ Da, or a mixture thereof.

28. Method according to claim 27 wherein the polymer is a hyaluronic acid salt of low molecular weight, where 0.5·10⁶ Da≤ M ≤0.75·10⁶ Da or a hyaluronic acid salt of high molecular weight (M), where 1.9·10⁶ ≤ M ≤2.2·10⁶ Da or a mixture thereof.

29. Method according to claims 27 or 28 wherein the hyaluronic acid salt is sodium hyaluronate.

30. Method according to claim 29 wherein the polymer solution comprises low molecular weight and/or high molecular weight sodium hyaluronate in a concentration between 0.1 and 3% (w/w), and preferably in a concentration between 0.5% and 2.5% (w/w) and most preferably in a concentration between 0.75 and 1.5% (w/w).

31. The method according to any of claims 15-30 wherein the aqueous solution medium used in step a) or i) is selected from:
- water,
- a buffer consisting of NaCl, in a concentration between 0.2 to 8%, and Na₂HPO₄ 12H₂O, in a concentration between 0.01% to 10% and NaH₂PO₄ 2H₂O, in a concentration between 0.001% and 10%, or
- a buffer consisting of sodium citrate dehydrate or a sodium citrate derivative, in a concentration between 0.002 to 2.5%, and citric acid (hydrated or dehydrated), in a concentration between 0.002% and 2%, and
- a buffer consisting of acetic acid in a concentration between 0.0005% and 0.1% and sodium acetate derivative in a concentration between 0.005% and 0.5%.

32. The method according to any claims 15-31 wherein the solvent used to dissolve the cannabinoid is defined by the formula: 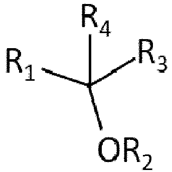 wherein R1-R4 are selected independently from H, OH, CH₂OH, CH₃, CH₂CH₃, C(O)CH₃, C(O)OCH₂CH₃, CH₂C(O)CH₂CH₃.

33. The method according to claim 32 wherein the solvent is selected from the group consisting of methanol, 1-propanol and isomers thereof, ethylene glycol, propylene glycol and mixtures thereof.

34. The method according to any one of claims 15-33 wherein the cross-linking agent is selected from a carbodiimide derivative, a carbonyl imidazole derivative, a carbonyl benzotriazole derivative, a carbonyl triazole derivative or mixtures thereof.

35. The method according to claim 34 wherein an active ester forming molecules is also present in the cross-linking step, preferably, sulfo hydroxisuccinimide (sulfo-NHS).

36. Method according to any one of claims 34-35 wherein a dihydrazide derivative is also present in the cross-linking step d).

37. Method according to any one of claims 15-36 wherein, after the crosslinking step, a sterilization step is carried out.

38. Method, according to claim 37, wherein the sterilization process is performed by steam sterilization, in an autoclave at a temperature ranging from 120°C to 140°C, preferably at 121°C, for 15-20 minutes.

39. A pharmaceutical, cosmetic or nutraceutical composition comprising the controlled release system of cannabinoids formulation according to any one of claims 1-14.

40. A pharmaceutical composition, according to claim 39 for use in the treatment of inflammatory joint diseases.

41. A pharmaceutical composition according to claim 39 for the augmentation and/or repair of soft tissue and keratin materials.

42. A cosmetic composition according to claim 39 for use in the relaxation, calming and moisturization of the skin.

43. A nutraceutical composition according to claim 39 for use in relaxation, calming and moisturization of the skin and for the improvement of the well being of the human body.
